# EUROPEAN PATENT SPECIFICATION

(11) **EP 2 277 464 B1**
(45) Date of publication and mention of the grant of the patent: **04.04.2012**
(21) Application number: 10251317.3
(22) Date of filing: 23.07.2010
(51) Int. Cl.: A61B 17/34, A61M 25/06

(54) **Surgical port and frangible introducer assembly**
Chirurgischer Anschluss und zerbrechliche Einführanordnung
Port chirurgical et ensemble introducteur sécable

(30) Priority: 24.07.2009 US 228204 P; 05.05.2010 US 774183
(43) Date of publication of application: 26.01.2011
(73) Proprietor: Tyco Healthcare Group, LP, New Haven CT 06511 (US)
(72) Inventor: Fortier, Richard C., Concord, MA 01742 (US); Ransden, Jeffrey E., Fairfield, CT 06825 (US); Adams, Leland R., Ansonia, CT 06401 (US); Helfer, Joel N., Cheshire, CT 06410 (US); Bachman, Alan B., Milford, CT 06460 (US); Lehman, Adam I., Northford, CT 06472 (US)
(74) Representative: Soames, Candida Jane

(56) References cited:
- EP-A1- 2 044 889
- WO-A1-99/22804
- US-A1- 2008 048 011

## Description

### BACKGROUND

### Technical Field

The present disclosure relates generally to ports for use in minimally invasive surgical procedures, such as endoscopic and/or laparoscopic procedures, and more particularly, relates to an access port and an associated introducer to assist in deploying the port within a tissue tract of a patient.

### Description of Related Art

Minimally invasive surgery is a type of surgery performed through one or more small incisions in a patient's body, usually less than 25 mm (an inch) in diameter. Some potential advantages of minimal invasive surgery is that patients have less trauma to the body, lose less blood, have smaller surgical scars, and need less pain medication.

During a typical minimally invasive procedure, surgical objects, such as surgical access devices, e.g., trocar and cannula assemblies, or endoscopes, are inserted into the patient's body through the incision in tissue. In general, prior to the introduction of the surgical object into the patient's body, insufflation gas are used to enlarge the area surrounding the target surgical site to create a larger, more accessible work area. Accordingly, the maintenance of a substantially fluid-tight seal is desirable so as to prevent the escape of the insufflation gases and the deflation or collapse of the enlarged surgical site.

To this end, various ports with valves and seals are used during the course of minimally invasive procedures and are widely known in the art. EP 2 044 889 describes a portal (102) having at least one longitudinal port (106) for passage of a surgical object, the portal (102) comprising a compressible material and being adapted to transition from a first expanded condition to a second compressed condition. However, a continuing need exists for an access port and associated introducer, which can position the access port with relative ease and with minor inconvenience for the surgeon.

### SUMMARY

Accordingly, a surgical portal and introducer assembly includes an introducer dimensioned for at least partial positioning within a tissue tract, and having a longitudinal introducer channel extending therethrough and a portal positionable within the longitudinal channel of the introducer. The introducer defines leading and trailing ends, and further has a frangible segment adapted to separate to expose the introducer channel. The portal has at least one longitudinal port for passage of a surgical object. The portal comprises a compressible material and is adapted to transition from a first expanded condition to a second compressed condition upon advancement through the longitudinal channel of the introducer to facilitate passage through the introducer whereby, upon separating of the frangible segment, the portal is released from the longitudinal channel to transition toward the first expanded condition to be generally secured within the tissue tract.

The frangible segment may include a tear line defined along the wall of the introducer. A tether may be secured to the introducer adjacent the tear line with the tether being manipulated to cause tearing along the tear line. The introducer may define a generally tapered configuration where an internal dimension of the longitudinal introducer channel generally decreases from the trailing end thereof to the leading end thereof.

The portal may define leading and trailing ends. The at least one longitudinal port may extend between the leading and trailing ends and is adapted for reception of an object whereby compressible material defining the at least one port is adapted to deform to establish a substantial sealed relation with the object. The portal may include a plurality of longitudinal ports. The portal may comprise one of a foam material or a gel material.

### DESCRIPTION OF THE DRAWINGS

The above and other aspects, features, and advantages of the present disclosure will become more apparent in light of the following detailed description when taken in conjunction with the accompanying drawings in which:

**FIG. 1** is a side elevational view of the surgical portal and introducer assembly in accordance with the principles of the present disclosure;

**FIG. 2** is a side elevational view of the surgical portal of the assembly of **FIG. 1**;

**FIG. 3** is a side elevational view of the introducer of the assembly of **FIG. 1****;** and

**FIG. 4** is a partial cross-sectional view of another embodiment of the assembly.

### DETAILED DESCRIPTION

Particular embodiments of the present disclosure will be described herein with reference to the accompanying drawings. As shown in the drawings and as described throughout the following description, and as is traditional when referring to relative positioning on an object, the term "proximal" or "trailing" refers to the end of the apparatus that is closer to the user and the term "distal" or "leading" refers to the end of the apparatus that is further from the user. In the following description, well-known functions or constructions are not described in detail to avoid obscuring the present disclosure in unnecessary detail.

One type of minimal invasive surgery described herein is referred to as a single-incision laparoscopic surgery (SILS). SILS is an advanced minimally invasive surgical procedure, which would permit a surgeon to operate through a single entry point, typically the patient's navel. The disclosed SILS procedure involves insufflating the body cavity and positioning a portal member within, e.g., the navel of the patient. Instruments including an endoscope and additional instruments such as graspers, staplers, forceps or the like may be introduced within the portal member to carry out the surgical procedure.

The port assembly in the SILS procedure may be introduced into an incision with a Kelly clamp. However, the Kelly clamp may limit the surgeon's ability to properly place a SILS port due to the limited length of the Kelly clamp's arm and handle. Furthermore, visibility may become an issue due to the presence of the clamp and the surgeon's hand holding the clamp. Removal of the Kelly clamp subsequent to placement of the port may also present undesired obstacles.

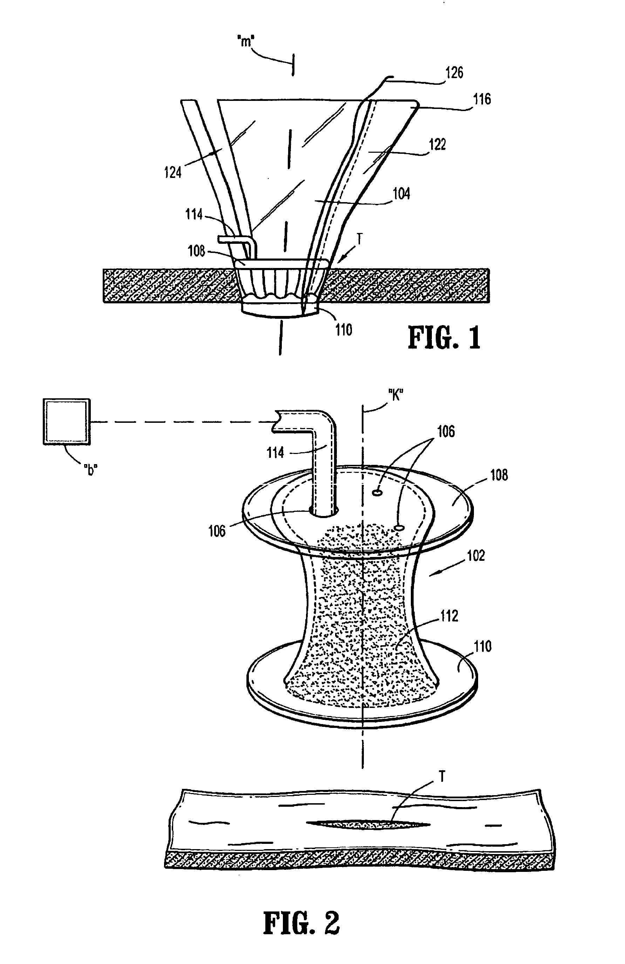

Referring now to the drawings, in which like reference numerals identify identical or substantially similar parts throughout the several views, **FIG.** 1 illustrates a surgical port and introducer assembly **100** including portal member **102** and introducer member 104 in accordance with the principles of the present disclosure. Portal member **102** is depicted enclosed within introducer member **104** in **FIG. 1****.** As best depicted in **FIG. 2****,** portal member **102** is adapted for insertion within a tissue tract **"T",** e.g., through the abdominal or peritoneal lining in connection with a laparoscopic surgical procedure. Portal member **102** includes at least one longitudinal port **106,** possibly, a plurality of longitudinal ports **106** extending along the axis **"k"** of the portal member **102.** At least one or more inner longitudinal ports **106** are dimensioned to receive a surgical object, e.g. a surgical instrument (not shown) therethrough. Upon introduction through a respective port **106,** the inner surface portions defining the port **106** establish and maintain a substantial sealed relation about the instrument or surgical object. Portal member **102** may define an hourglass shape as shown. Trailing and leading ends **108, 110** may define flange segments, which may be integrally formed with portal member **102.** Portal member 102 may be made from a disposable, compressible, and/or flexible type material, for example, but not limited to, a suitable foam or gel material having sufficient compliance to form a seal about one or more surgical objects, and also establish a sealing relation with the tissue. The foam is preferably sufficiently compliant to accommodate off axis motion of the surgical object. In one embodiment, the foam includes a polyisoprene material. When inserted within the tissue tract **"T",** portal member **102** is adapted to establish a substantial seal within the tract **"T",** i.e., with the tissue surfaces defining the tract **"T".** During insertion, portal member 102 may be compressed to a compressed condition to permit at least partial passage through the tract **"T".** Once within the tract **"T",** portal member **102** will return toward the normal expanded condition with the outer wall **112** of the portal member **102** establishing a seal with the tissue defining the tissue tract **"T".** Portal member **102** may include an insufflation conduit **114** mounted within one of passageways **106** and connectable to a source of insufflation gases to permit passage of gases, **CO2,** to maintain the pneumoperitoneum. Suitable portal members **102** are disclosed in commonly assigned U.S. patent application Serial No. 12/244,024, filed October 2, 2008**,** the entire contents of which is hereby incorporated by reference herein.

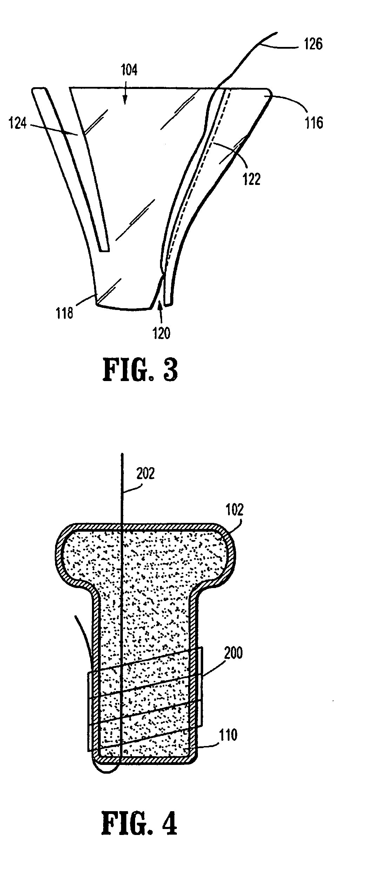

With reference to **FIGS.** 1 and **3****,** introducer member **104** is adapted to facilitate insertion of portal member **102** within the tissue tract **"T".** Introducer member **104** is substantially elongated defining longitudinal axis **"m"** and trailing or proximal end **116** and leading or distal end **118.** Introducer member **102** defines a longitudinal introducer channel **120** for reception and passage of portal member 102. In one embodiment, introducer member 102 defines a generally tapered or funnel shaped configuration having an internal dimension which decreases from proximal to distal. The relatively narrow configuration of leading or distal end **118** facilitates insertion within tissue tract **"T".** Introducer member **104** may be made of any type of suitable material, for example, but not limited to, a polymeric material, and may be rigid or flexible.

Introducer member **104** includes a frangible tear segment or line **122** extending along the wall of the introducer member **102.** Frangible tear segment **122** may include a perforated or score line or may incorporate a weakened section in the wall of introducer member **104.** Introducer member **104** is adapted to tear along the tear line **122** to permit removal of the introducer member **104** subsequent to insertion of portal member **102** within the tissue tract **"T".** Frangible tear segment **122** is oriented along a wall of the introducer member **104** between the proximal end **116** and the distal leading end **118.** Introducer member **104** may further include slit **124** that is defined along the wall of the introducer member **104.** Slit **124** is dimensioned to permit insufflation conduit **114** of the portal member 102 to pass therethrough as the portal member **102** is advanced through introducer member **104.**

A tether **126** may be attached adjacent frangible segment or line **122** and extending toward distal leading end **118** of introducer member **104.** Tether **126** may be made of a material, such as, wire, suture, or shape-memory alloy. Tether **126** is adapted to separate the wall of the introducer **104** along the frangible tear line **122** as the tether **126** is selectively pulled in a proximal trailing direction.

In embodiments of the present disclosure, surgical portal and introducer **100** may come preassembled with portal member **102** disposed within introducer member **104.** In the alternative, portal member **102** may be positioned within introducer member **104** at the surgical theatre or site.

A method of introducing and deploying portal member **102** includes positioning leading or distal end **118** of introducer member **104** within the tissue tract **"T"** and advancing the leading end **118** to a predetermined depth. It is envisioned that the introducer member **104** may be made from a translucent-type material such that the clinician may monitor the depth the introducer/portal combination is being deployed within a tissue tract **"T".** Thereafter, portal member **102** is positioned within proximal or trailing end **116** of introducer member **104** (if not preassembled as hereinabove discussed). Upon insertion, portal member **102** compresses to fit within the inner boundary of introducer channel **120** of introducer member **104.** Portal member **102** is advanced relative to tissue tract "T" by either advancing the portal member **102** within introducer member **104** or advancing the introducer member **104** further into the tract **"T".** During advancement, insufflation conduit **114** of portal member **102** may traverse slit **124** in the wall of introducer member **104.** Once portal member **102** is located within the tissue tract **"T",** e.g., with leading and trailing ends **118, 116** of the portal member **102** on opposed sides of the body wall (e.g., the abdominal cavity wall), introducer member **104** is removed by pulling tether in a general proximal direction or radial outward direction relative to longitudinal axis **"m"** to effect tearing of the introducer member **104** along frangible segment **122.** Introducer member **104** is removed and portal member **102** expands toward its normal expanded condition in sealed engagement with the tissue defining the tissue tract.

In another embodiment illustrated in **FIG. 4****,** a portal member **102** is shown wrapped with a suitable band material **200,** for example, but not limited to, a tape material or a thin plastic material. Band material **200** may be kept intact by an adhesive and/or hook and loop fastener, such as VELCRO. At least a length (or, possibly, the entire length) of portal member **102** may be wrapped by the band material **200** such that portal member **102** is compressed. After portal member **102** is wrapped and compressed by band material **200,** the portal member **102** is inserted into a tissue tract **"T"** of a patient at a desired depth. It is envisioned that a thin tether **202,** e.g., wire or suture, is disposed within the band material **200.** In this configuration, when the tether **202** is pulled by a clinician, it results in cutting and tearing the band material **200** to release portal member **102** to permit the portal member **102** to assume or move toward an expanded state. In embodiments, band material **200** may be perforated.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. A surgical portal and introducer assembly (100), which comprises:
an introducer (104) dimensioned for at least partial positioning within a tissue tract, the introducer (104) defining a longitudinal axis and having a longitudinal introducer channel (120) extending therethrough, the introducer (104) defining leading (118) and trailing (116) ends, the introducer (104) includes a frangible segment (122) adapted to separate to expose the introducer channel (120); and
a portal (102) positionable within the longitudinal channel (120) of the introducer (104), the portal (102) having at least one longitudinal port (106) for passage of a surgical object, the portal (102) comprising a compressible material and being adapted to transition from a first expanded condition to a second compressed condition upon advancement through the longitudinal channel (120) of the introducer (104) to facilitate passage through the introducer (104) whereby, upon separating of the frangible segment (122), the portal (102) is released from the longitudinal channel (120) to transition toward the first expanded condition to be generally secured within the tissue tract.

2. The surgical assembly (100) according to claim 1, wherein the frangible segment (122) includes a tear line defined along the introducer (104).

3. The surgical assembly (100) according to claim 2, including a tether (126) secured to the introducer (104) adjacent the tear line, the tether (126) being manipulable to cause tearing along the tear line.

4. The surgical assembly (100) according to claim 2, wherein the introducer (104) defines a generally tapered configuration where an internal dimension of the longitudinal introducer channel (120) generally decreases from the trailing (116) end thereof to the leading (118) end thereof.

5. The surgical assembly (100) according to any preceding claim, wherein the portal (102) defines leading (110) and trailing (108) ends, the at least one longitudinal port (106) extending between the leading (110) and trailing (108) ends and being adapted for reception of an object whereby compressible material defining the at least one port (106) is adapted to deform to establish a substantial sealed relation with the object.

6. The surgical assembly (100) according to claim 5, wherein the portal (102) includes a plurality of longitudinal ports (106).

7. The surgical assembly (100) according to any preceding claim, wherein the portal (102) comprises one of a foam material or a gel material.

## Patentansprüche

1. Chirurgische Zugangs- und Einführungsanordnung (100), die Folgendes umfasst:
eine Einführungsvorrichtung (104), die abgemessen ist, um mindestens teilweise in einem Gewebetrakt angeordnet zu sein, wobei die Einführungsvorrichtung (104) eine Längsachse definiert und einen Längs-Einführungskanal (120) umfasst, der sich **dadurch** erstreckt, wobei die Einführungsvorrichtung (104) ein vorderes (118) und ein hinteres (116) Ende definiert, wobei die Einführungsvorrichtung (104) einen zerbrechlichen Abschnitt (122) umfasst, der dazu ausgelegt ist, um zu trennen, um den Einführungskanal (120) auszusetzen; und
eine Zugangsvorrichtung (102), die innerhalb des Längskanals (120) der Einführungsvorrichtung (104) angeordnet werden kann, wobei die Zugangsvorrichtung (102) mindestens einen Längszugang (106) zum Durchgang eines chirurgischen Gegenstands aufweist, wobei die Zugangsvorrichtung (102) ein komprimierbares Material aufweist und dazu ausgelegt ist, beim Vorrücken durch den Längskanal (120) der Einführungsvorrichtung (104) von einem ersten ausgedehnten Zustand in einen zweiten, komprimierten Zustand überzugehen, um den Durchgang durch die Einführungsvorrichtung (104) zu erleichtern, wodurch, bei der Trennung des zerbrechlichen Abschnitts (122), die Zugangsvorrichtung (102) vom Längskanal (120) freigegeben wird, um zum ersten, ausgedehnten Zustand überzugehen, um im Allgemeinen innerhalb des Gewebetrakt befestigt zu sein.

2. Chirurgische Anordnung (100) nach Anspruch 1, wobei der zerbrechliche Abschnitt (122) eine Reißlinie umfasst, die entlang der Einführungsvorrichtung (104) definiert ist.

3. Chirurgische Anordnung (100) nach Anspruch 2, umfassend einen Gurt (126), der an die Einführungsvorrichtung (104) nahe an der Reißlinie befestigt ist, wobei der Gurt (126) betätigt werden kann, um das Reißen entlang der Reißlinie hervorzurufen.

4. Chirurgische Anordnung (100) nach Anspruch 2, wobei die Einführungsvorrichtung (104) eine im Allgemeinen konische Konfiguration definiert, bei der eine innere Dimension des Längs-Einführungskanals (120) im Allgemeinen vom hinteren (116) Ende davon zum vorderen Ende (118) davon abnimmt.

5. Chirurgische Anordnung (100) nach einem der vorhergehenden Ansprüche, wobei die Zugangsvorrichtung (102) ein vorderes (110) und ein hinteres (108) Ende definiert, wobei sich der mindestens eine Längszugang (106) zwischen dem vorderen (110) und dem hinteren (108) Ende erstreckt und dazu ausgelegt ist, um einen Gegenstand aufzunehmen, wodurch komprimierbares Material, das den mindestens einen Zugang (106) definiert, dazu ausgelegt ist, sich zu deformieren, um eine im Wesentlichen versiegelte Verbindung mit dem Gegenstand herzustellen.

6. Chirurgische Anordnung (100) nach Anspruch 5, wobei die Zugangsvorrichtung (102) eine Vielzahl von Längszugängen (106) umfasst.

7. Chirurgische Anordnung (100) nach einem der vorhergehenden Ansprüche, wobei die Zugangsvorrichtung (102) entweder ein Schaummaterial oder ein Gelmaterial umfasst.

## Revendications

1. Ensemble port chirurgical et introducteur (100), qui comprend :
un introducteur (104) dimensionné pour un positionnement au moins partiel à l'intérieur d'un tractus tissulaire, l'introducteur (104) définissant un axe longitudinal et ayant un canal introducteur longitudinal (120) s'étendant à travers lui, l'introducteur (104) définissant des extrémités avant (118) et arrière (116), l'introducteur (104) comprenant un segment sécable (122) adapté pour se séparer pour exposer le canal introducteur (120) ; et
un port (102) positionnable à l'intérieur du canal longitudinal (120) de l'introducteur (104), le port (102) ayant au moins un orifice longitudinal (106) pour le passage d'un objet chirurgical, le port (102) étant constitué d'une matière compressible et étant adapté pour passer d'un premier état dilaté à un second état comprimé lors de la progression à travers le canal longitudinal (120) de l'introducteur (104) pour faciliter le passage à travers l'introducteur (104), moyennant quoi, lors de la séparation du segment sécable (122), le port (102) est libéré du canal longitudinal (120) pour passer au premier état dilaté pour être généralement fixé à l'intérieur du tractus tissulaire.

2. Ensemble chirurgical (100) selon la revendication 1, dans lequel le segment sécable (122) comprend une ligne de séparation le long de l'introducteur (104).

3. Ensemble chirurgical (100) selon la revendication 2, comprenant une attache (126) fixée à l'introducteur (104) de façon adjacente à la ligne de séparation, l'attache (126) étant manipulable pour entraîner une séparation le long de la ligne de séparation.

4. Ensemble chirurgical (100) selon la revendication 2, dans lequel l'introducteur (104) définit une configuration généralement effilée où une dimension interne du canal introducteur longitudinal (120) diminue généralement de l'extrémité arrière (116) de celui-ci vers l'extrémité avant (118) de celui-ci.

5. Ensemble chirurgical (100) selon l'une quelconque des revendications précédentes, dans lequel le port (102) définit des extrémités avant (110) et arrière (108), l'au moins un orifice longitudinal (106) s'étendant entre les extrémités avant (110) et arrière (108) et étant adapté pour recevoir un objet, moyennant quoi un matériau compressible définissant l'au moins un orifice (106) est adapté pour se déformer pour établir une relation sensiblement étanche avec l'objet.

6. Ensemble chirurgical (100) selon la revendication 5, dans lequel le port (102) comprend une pluralité d'orifices longitudinaux (106).

7. Ensemble chirurgical (100) selon l'une quelconque des revendications précédentes, dans lequel le port (102) comprend l'une d'une matière de type mousse ou d'une matière de type gel.
